(19) Europäisches Patentamt
European Patent Office
Office européen des brevets

(11) Publication number: 0 219 144
B1

# (12) EUROPEAN PATENT SPECIFICATION

(45) Date of publication of the patent specification: 27.12.89

(51) Int. Cl.⁴: C 07 D 307/88, A 01 N 43/12

(21) Application number: 86201428.9

(22) Date of filling: 15.08.86

(54) **Diphenyl ether herbicides.**

(30) Priority: 11.09.85 GB 8522528

(43) Date of publication of application: 22.04.87 Bulletin 87/17

(45) Publication of the grant of the patent: 27.12.89 Bulletin 89/52

(84) Designated Contracting States: AT BE CH DE FR GB IT LI LU NL SE

(56) References cited:
EP-A-0 145 078

CHEMICAL ABSTRACTS, vol. 84, no. 5, 2nd February 1976, page 386, column 2, abstract no. 30115t, Columbus, Ohio, US; M. ROZE et al.: "Carboxylic acids of diphenyl oxide. I. Liquid-phase catalytic oxidation of 3,4-dimethyldiphenyl oxide", & LATV. PSR ZINAT. AKAD. VESTIS, KIM. SER. 1975, (5), 594-599 in connection with Ninth Collective Index, CHEMICAL ABSTRACTS, Chemical Substances, vol. 76-85, 1972-1976, page 21038CS, column 1, "5-phenoxy-1(3H)-isobenzofuranone"

The file contains technical information submitted after the application was filed and not included in this specification

(73) Proprietor: SHELL INTERNATIONALE RESEARCH MAATSCHAPPIJ B.V., Carel van Bylandtlaan 30, NL- 2596 HR Den Haag (NL)

(72) Inventor: Clark, Michael Thomas, Torcross Wises Lane, Borden Sittingbourne, Kent (GB)

(74) Representative: Bennett, David Arthur Horder, 4, York Road, London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

EP 0 219 144 B1

LIBER, STOCKHOLM 1989

## Description

This invention relates to certain diphenyl ether derivatives, the preparation of such compounds, herbicidal compositions containing them, and to their use in combating undesired plant growth.

Applicants' European Patent Application No. 145 078 describes and claims a herbicidal composition which comprises a carrier and, as active ingredient, a diphenyl ether derivative having the general formula I:

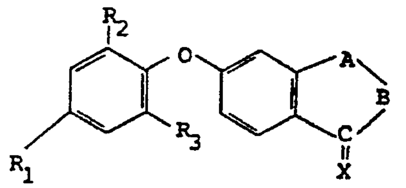

(I)

wherein $R_1$ represents a hydrogen or halogen atom or an alkyl or haloalkyl group; $R_2$ and $R_3$, which may be the same or different, each independently represents a hydrogen or halogen atom or an alkyl, haloalkyl, nitro or cyano group; and B and A, inter alia, represent, respectively, an oxygen atom and a group of formula $=C(R_4)2$ in which each $R_4$, which may be the same or different, represents a hydrogen or halogen atom or an optionally substituted alkyl, cycloalkyl, alkenyl, alkynyl, aryl, aralkyl, alkaryl, alkoxy, cycloalkoxy, alkenyloxy, alkynyloxy, alkoxycarbonylalkoxy, alkylthio, acyl, acyloxy, carboxyl, alkoxycarbonyl or heterocyclic group, an amino group of formula $NR_6R_7$, or when one $R_4$ represents a hydrogen atom, an alkoxy group or a hydrocarbon group, then the other may represent a hydroxyl group, or both groups $R_4$ together may represent an imino group of formula $=NR_6$;

$R_5$ represents a hydrogen atom, a carboxyl or hydroxyl group, or an optionally substituted alkyl, alkoxy, aralkyl, alkoxycarbonyl or alkoxycarbonylalkyl group, or an amino group of formula $NR_6R_7$;

$R_6$ and $R_7$, which may be the same or different, each represents a hydrogen atom or an optionally substituted alkyl, aryl or acyl group;

and X, both in formula I and in the definitions of the substituents therein, represents an oxygen or sulphur atom.

The normal agronomic application of herbicides involves spraying them onto crop areas, either before or after emergence of the crop according to requirement. After spraying, the herbicide is then, of course, exposed to moisture present in the soil and to normal rainfall, and it is therefore of great practical importance that the hydrolytic stability of the active ingredient should be sufficient to prevent premature loss of herbicidal efficacy. It has now been found, unexpectedly, that a certain group of the compounds broadly described in EP-A-145 078, but not specifically disclosed therein, possess a significantly higher hydrolytic stability then the generality of the diphenyl ether phthalides actually disclosed. These compounds are characterised by the presence at substituent location A of an alkylidene group of formula

\
C(alkyl)$_2$.
/

Dialkyl phthalides of this type, as a consequence of this unexpected higher hydrolytic stability, also demonstrate a higher level of herbicidal activity in actual field applications compared with the generality of the diphenyl ether phthalides actually disclosed in EP-A-145 078.

Accordingly, the present invention provides phenoxy phthalide derivatives having the general formula II:

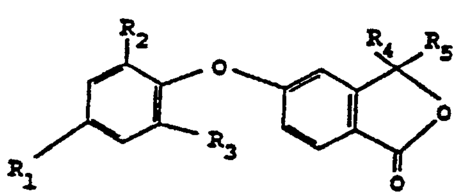

(II)

wherein $R_1$ represents a hydrogen or halogen, preferably chlorine, atom or an alkyl or haloalkyl, preferably trifluoromethyl, group; $R_2$ and $R_3$, which may be the same or different, each independently represents a hydrogen or halogen, preferably chlorine, atom or an alkyl, haloalkyl, preferably trifluoromethyl, nitro or cyano group; and $R_4$ and $R_5$, which may be the same or different, each independently represents an alkyl group.

When any of the foregoing substituents represents or contains an alkyl substituent group, this may be linear or branched and may contain up to 10, preferably up to 6, carbon atoms, suitable examples being methyl and ethyl. When they contain a haloalkyl substituent group, this suitably contains up to 6, preferably up to 4, carbon atoms and the halogen atom is suitably fluorine or chlorine, trifluoromethyl being particularly preferred. When any of the foregoing substituents are designated as being optionally substituted, the substituent groups which are optionally present may be any of those customarily employed in the development of pesticidal compounds, and/or the modification of such compounds to influence their structure/activity, persistence, penetration or other

property. Specific examples of such substituents include halogen, especially chlorine, atoms and nitro, cyano, alkyl or haloalkyl, especially trifluoromethyl, groups.

Preferred compounds are those wherein $R_1$ represents a halogen, more particularly chlorine, atom or, especially, a trifluoromethyl group; $R_2$ represents a nitro, cyano, trifluoromethyl or, especially, a halogen, more particularly chlorine, atom; and $R_3$ represents a halogen, more particularly chlorine, or, especially, a hydrogen atom.

$R_4$ and $R_5$ are preferably independently selected from alkyl groups of 1 - 6 carbon atoms, especially those of 1 - 4 carbon atoms. More preferably still, $R_4$ and $R_5$ are independently selected from methyl and ethyl. One may advantageously be methyl and the other ethyl.

It will be appreciated that when the nature of the substituents is such as to introduce an asymmetric carbon atom, then the resulting compound will exist in stereisomeric forms. Also, some substituent combinations permit the existence of tautomeric forms. Often, one of these isomeric forms will have greater biological activity than other forms. The scope of the present invention includes these different forms of the compounds and their mixtures, and herbicidal compositions containing the active ingredient in such isomeric forms and mixtures.

The invention also provides a process for the preparation of a phenoxy phthalide derivative of formula II as claimed in any of claims 1 to 6, which comprises reacting a compound of formula III

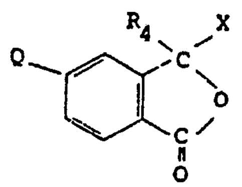

(III)

where X represents a hydrogen atom, a hydroxy group or a complex organometallic alkoxide group, Q represents a halogen atom or a nitro group or a group of formula -OZ, where Z represents a hydrogen or alkali metal atom or a group of formula

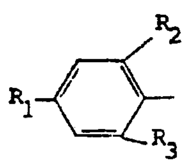

with a compound of formula $R^5$-$(M)_p$-Hal (IV) where M represents a metal atom, p is O (when X is hydrogen) or 1 (when X is other than hydrogen) and Hal represents a halogen atom, and, when the product is a compound in which Q does not represent a group of formula

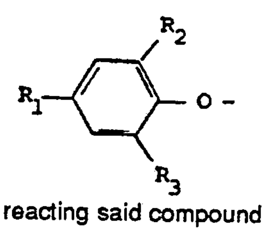

reacting said compound with a compound of formula V

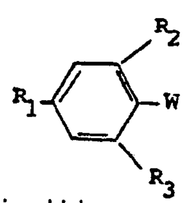

(V)

in which compound W represents a halogen atom or a nitro group when Q represents a group -OZ where Z represents a hydrogen or alkali metal atom, and W represents such a group -OZ when Q represents a halogen atom or a nitro group.

The moiety Hal is preferably a bromine or iodine atom.

When p is O, the reaction is carried out in the presence of a base, preferably a strong base, and an aprotic solvent. Suitable bases include alkali amines such as sodamide and lithium dialkylamines, for example lithium diisopropylamine and lithium diethylamine, alkali metal alkoxides such as potassium tert-butyloxide and sodium tert-pentoxide, lithium N-isopropyl-N-cyclohexylamide, triphenylmethylsodium and butylmagnesium bromide. Suitable solvents include 1.2-dimethoxyethane, dimethylformamide, tetrahydrofuran, liquid ammonia, dimethyl sulphoxide, acetonitrile, acetone, nitrobenzene, hexamethylphosphoramide and hexane. The reaction preferably takes place at a temperature below ambient temperature, suitably in the range -100 to 0°C.

When p is 1 the organometallic reagent used is preferably an organomagnesium compound (Grignard reagent), prepared according to established procedures, e.g. by taking up the appropriate alkyl halide and magnesium metal in an aliphatic ether, such as diethyl ether, in the absence of water. The reaction of the compound of formula III with that Grignard reagent is suitably carried out in a solvent, which may also be diethyl ether, or may be a different inert organic solvent such as tetrahydrofuran. The formation of the Grignard reagent and its reaction with the compound of formula III are each suitably carried out in the temperature range 0 to 50°C, preferably at ambient temperature. The Grignard organomagnesium complex may be supplemented by the generation of an organocadmium complex through the addition of cadmium chloride.

When the above reactions are effected to make compounds in which Q is other than a group

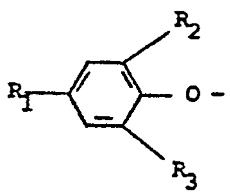

that is to say, compounds in which one of W and Q represents a halogen, suitably chlorine, atom or nitro group and the other represents a group of formula -OZ where Z represents a hydrogen atom or alkali metal atom, it is preferably the moiety W which represents the halogen atom or nitro group. The reaction is conveniently carried out by reacting a compound of formula V wherein W represents a halogen, suitably chlorine, atom or a nitro group with an alkali metal alkoxide compound of formula III produced by reacting the corresponding compound in which Q represents hydroxy, with an alkali metal hydroxide in an alkanol solvent, for example, ethanol. The reaction of the alkoxide with the compound V is preferably carried out in a suitable solvent, for example, dimethyl sulphoxide, sulpholane, dimethyl formamide, or dimethyl acetamide at elevated temperature, conveniently under reflux, and also under an inert atmosphere such as nitrogen.

It is preferred, however, to effect the above reactions with starting materials in which Q represents a group

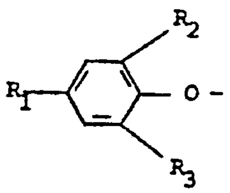

so that a further step to produce compounds of formula II is not required.

The compounds of formula III are preferably prepared by reactions with organometallic reagents under conditions as set forth above. The compounds of formula III wherein X is hydrogen are suitably prepared by reaction of corresponding compounds wherein $R_4$ is a hydroxy group, with a compound $R_4$-M-Hal where $R_4$ and Hal are as described above. The final step to introduce the group $R_5$ may then be effected. This group $R_5$ may be different from or the same as the group $R_4$. However, it will normally be different since compounds of formula II wherein $R_4$ and $R_5$ are identical may readily be prepared in one pot by reaction of the corresponding phthalic anhydride with a compound $R_4$-M-Hal. The initial product, the compound of formula III wherein X represents a group -O-M-Hal (or, if acidified, a hydroxy group) reacts *in situ* with a further organometallic species.

Since the phthalic anhydride starting material mentioned above contains two ring carbonyl groups, the Grignard reagent may react with either of them. In practice, it is usually found that the major product is the desired phthalide of formula II, with the isomeric phthalide of formula VI below being formed as a minor by-product

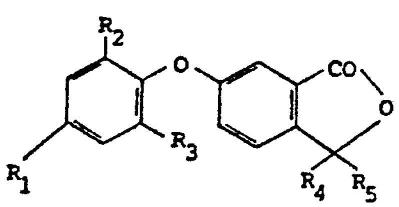

(VI)

It will be apparent from the foregoing that the preferred method for preparing the compounds of formula II wherein $R_4$ and $R_5$ are identical is to react a corresponding phthalic anhydride with a Grignard reagent. The preferred method for preparing the compounds of formula II in which $R_4$ and $R_5$ differ is to react the corresponding compound having the group

```
H OH
 \ /
  C
 / \
```

with a Grignard reagent to produce the corresponding compound having the group

```
H  R4
 \ /
  C
 /\
```

and reacting that compound with $R_5$-Hal, where $R_5$ is different from $R_4$.

The phenoxy phthalides of this invention may be converted to the corresponding thiophthalides by reaction with phosphorus pentasulphide, suitably in an inert organic solvent such as dioxan. These thiophthalides share the enhanced hydrolytic stability of the $=C(alkyl)_2$ compounds of this invention, in that they are hydrolytically more stable than analogous thiophthalides in which $R_4$ is a hydrogen atom; however, they are intrinsically less stable than the phthalides. In fact, hydrolysis of the thiophthalides results in the generation of the corresponding phthalide compounds in substantially quantitative yield.

The compounds of general formula II have been found to show, interesting activity as herbicides. Accordingly, the invention further provides a herbicidal composition comprising a compound of formula II as defined above in association with at least one carrier, and a method of making such a composition which comprises bringing a compound of formula II into association with at least one carrier.

The invention also provides the use of such a compound or composition according to the invention as a herbicide. Further, in accordance with the invention there is provided a method of combating undesired plant at a locus by treating the locus with a compound or composition according to the invention. Application to the locus may be pre-emergence or post-emergence. The dosage of active ingredient used may, for example, be from 0.05 to 4 kg/ha. A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95 % by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate synthetic hydrated silicon oxides and synthetic calcium or aluminum silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75 % w of active ingredient and usually contain in addition to solid inert carrier, 3 - 10 % w

of a dispersing agent and, where necessary, 0 - 10 % w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing 1/2 - 10 % w of active ingredient. Granules are usually prepared to have a size between 10 and 100 BS mesh (1.676 - 0.152 mm), and may be manufactured agglomeration or impregnation techniques. Generally, granules will contain 1/2 - 75 % w active ingredient and 0 - 10 % w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10 - 50 % w/v active ingredient, 2 - 20 % w/v emulsifiers and 0 - 20 % w/v of other additives such as stabilisers, penetrants and corrosion inhibitors. Suspension concentrates are usually compounded so as to obtain a stable, non-sedimenting flowable product and usually contain 10 - 75 % w active ingredient, 0.5 - 15 % w of dispersing agents, 0.1 - 10 % w of suspending agents such as protective colloids and thixotropic agents, 0 - 10 % w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain otter ingredients, for example other compounds possessing herbicidal, insecticidal or fungicidal properties.

The invention is illustrated in the following Examples.

**Example 1**

**5-(2'-Chloro-4'-trifluoromethylphenoxy)-3,3-dimethyl phthalide**

Dry magnesium turnings (1.5 g) were added to sodium dried diethyl ether (100 ml), and iodomethane (10 g) added dropwise with vigorous stirring under dry nitrogen. After 1/2 hour anhydrous cadmium chloride (11.0 g) was added in portions, stirred for 45 minutes and the reaction mixture cooled to 0°C. 5-(2-chloro-4-trifluoromethyl phenoxy) phthalic anhydride (10.3 g) dissolved in dry diethyl ether (60 ml) was added dropwise with vigorous stirring, the mixture allowed to warm to room temperature and then refluxed for 17 hours. 10 % sulphuric acid (100 ml) was added, and the resulting clear solution extracted with diethyl ether. The extracts were washed, dried, the solvent removed, and the product chromatographically purified to yield the title compound as a white solid, 1.45 g, m.pt. 88 - 92°C

| | | |
|---|---|---|
| Analysis: | Calc. for $C_{17}H_{12}ClF_3O_3$: | C 57.2; H 3.4 % |
| | Found: | C 57.1; H 3.5 % |

| | |
|---|---|
| N.M.R. results:- | delta 7.84 - 6.92 ppm 6H (aromatics) |
| | delta 1.64 ppm singlet 6H (methyl groups) |

A similar, larger-scale method which did not employ cadmium chloride gave a yield of 33.5 g of the desired compound from 34.25 g of the starting material.

**Example 2**

**5-(2'-Chloro-4'-trifluoromethylphenoxy)-3,3-diethylphthalide**

3M ethyl magnesium bromide in ether (41.5 ml) was syringed through a septum cap under dry nitrogen into a flask, to which dry diethyl ether (85 ml) and 5-(2-chloro-4-trifluoromethyl phenoxy) phthalic anhydride (5.7 g) dissolved in dry tetrahydrofuran (50 ml) were added dropwise with stirring at room temperature. The mixture was stirred for 1/2 hr, poured onto ice/concentrated hydrochloric acid, and extracted with diethyl ether. The ether extracts were dried and chromatographically purified to yield the title compound (second, fraction, solid, 4.7 g, m.p., 57°C), and

| | | |
|---|---|---|
| Analysis: | Calc: | C 59.3; H 4.2 |
| | Found: | C 59.2; H 3.8 % |

| | |
|---|---|
| N.M.R. results delta | 7.78 - 6.82 ppm (6H) |
| | 2.08 sextuplet (doublet of quartets) (2H) |
| | 1.88 sextuplet (doublet of quartets) (2H) |
| | 0.72 triplet (6H) |

**Example 3**

**5-(2',6'-Dichloro-4'-trifluoromethylphenoxy)-3,3-dimethyl phthalide**

The title compound, m.p. 89 - 92°C, was prepared by the method of Example 1.

| | | |
|---|---|---|
| Analysis: | Calc: | C 52.5; H 2.8 |
| | Found: | C 51.8; H 2.9 |

| | |
|---|---|
| N.M.R. results delta | 7.8 - 6.82 ppm multiplet (5H) |
| | 1.64 singlet (6H) |

**Example 4**

**5-(2'-Chloro-4'-trifluoromethylphenyl)-3-methyl-3-ethyl phthalide**

3M Methyl magnesium bromide in diethyl ether (41.5 ml) was added to diethyl ether (84 ml) in a three-necked roundbottomed flask, through a septum cap by syringe under dry nitrogen. The solution was vigorously stirred whilst a solution of 5-(2'-chloro-4'-trifluoromethyl-phenoxy)-3-hydroxy phthalide (5.7 g) in dry tetrahydrofuran (50 ml) was added drop by drop. The temperature was maintained below the reflux temperature. After addition was complete the reaction mixture was poured into ice (500 g) containing concentrated hydrochloric acid (20 ml), and was extracted with diethyl ether. The ether extracts were dried, the solvent removed by evaporation and the residue chromatographically purified to yield (2'-chloro-4'-trifluoromethylphenoxy)-3-methyl phthalide, m.p. 107°C, yield 3.95 g.

| | | |
|---|---|---|
| Analysis: | Caltulated: | 56.1 % C 2.9 % H |
| | Found: | 56.3 % C 3.2 % H |

4.0 ml of 1.6 n-butyl lithium in hexane was mixed at -78°C with diisopropylamine (0.8 ml) in dry tetrahydrofuran (30 ml). The product of the previous step (2.0 g) in dry tetrahydrofuran (20 ml) was added dropwise and stirred at -78°C for 2 minutes Iodoethane (4 ml) was added by syringe. The reaction mixture was stirred overnight, poured into 50 % hydrochloric acid, extracted with dichloromethane, washed, dried, and purified by chromatography. The main fraction, a pale green-yellow oil, was the title compound (1.55 g)

| | | |
|---|---|---|
| Analysis: | Calculated: | 58.3 % C 3.8 % H |
| | Found: | 56.8 % C 4.0 % H |

| | |
|---|---|
| N.M.R. results delta | 7.85-6.85 ppm, multiplet (6H) |
| | 2.05 sextuplet (1H) |
| | 1.85 sextuplet (1H) |
| | 1.6 singlet (3H) |
| | 0.75 triplet (3H) |

**Example 5**

**Hydrolytic Stability**

The hydrolytic stability of the compounds of this invention was compared with representative examples of other phenoxy phthalides specifically described in Applicants' earlier EP-A-145 078. This property was evaluated by determining the half-life (in hours) at pH 7.0 and 9.0 in 25 % 1,4-dioxan at 39°C. The results of these tests are set out in Table I below, in which the test compounds are identified by reference to the substituent A in the following formula:

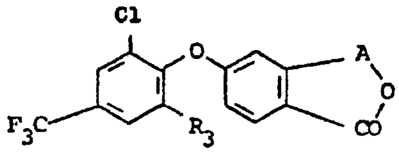

**Table 1**

| A | | B3 pH 7.0 | Half-Life pH 9.0 |
|---|---|---|---|
| $CH_2$ | H | 56.5 | 14.5 |
| $CH(OCH_3)$ | H | 17.2 | 1.2 |
| $CH(CH_3)$ | H | 1057 | 11.0 |
| $C(CH_3)_2$ | H | 4915 | 1904 |
| $C(C_2H_5)_2$ | H | NDD | NDD |
| $C(CH_3)(C_2H_5)$ | H | NDD | NDD |
| $C(CH_3)_2$ | Cl | NDD | NDD |

'N.D.D.' = No detectable decomposition over a 200 hour period

These results clearly demonstrate that the compound wherein A represents $C(alkyl)_2$, i.e. the compounds of this invention, possess a considerably greatly hydrolytic stability, especially under alkaline condition, than those analogous compounds having one or two hydrogen atoms on that carbon atom.

**Example 6**

**Herbicidal Activity**

Trials were carried out to compare the herbicidal activity in the field between a compound of the invention (A) and a representative of the compounds specifically described in EP-A-145 078.

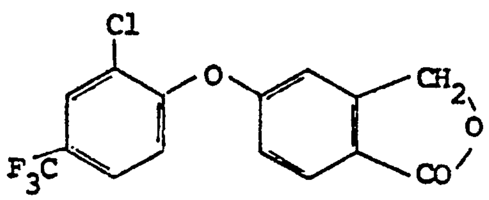

(B)

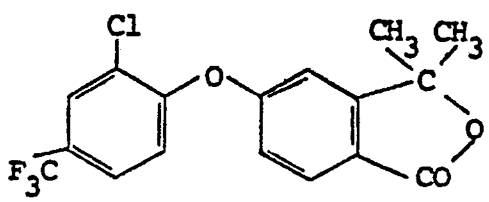

(A)

The trials were carried out on winter wheat, using 3 x 8 m plots in randomised blocks or semi-balanced lattices. The test compounds were applied post emergence at a spray volume of 400 l/ha, and weed assessments made on a % cover basis approximately six weeks after treatment. The overall effect against broad leaved weeds is determined by summing the amounts of individual weeds present in the experimental plots and comparing this with the total % cover of all broad leaved weed plants in the untreated controls. This figure is designated TOTDI.

The results of these trials are sat out in Table 2 below, in which the weed species are designated by the abbreviations:

STEME = Stellaria Media
MATSS = Matricaria Spp.
BRANA = Brassica Napus
POLAN = Polygonum Aviculare
VERPE = Veronica Persica
LAMPU = Lamium Purpurea
VIOAR = Viola Arversis
MYOAR = Myosotis Arvensis
APHAR = Aphanes Arvensis
RANAR = Ranunculus Arvensis

PAPRH = Papaver Rhoeas
CAPBP = Capsella Bursa-pastoris
VERHE = Veronica Hederifolia

**Table 2**

| COMPOUND | DOSE kg/ha | STEME | MATSS | BRANA | POLAV | VERPE | LAMPU | VIOAR | MYOAR | APHAR | RANAR | PAPRH | CAPBP | VERHE | TOTDI |
|---|---|---|---|---|---|---|---|---|---|---|---|---|---|---|---|
| A | 0.100 | 77 | 76 | 80 | 100 | 100 | | | | | | | | | 78 |
| | | 60 | 48 | | 100 | 100 | 65 | 98 | 14 | 33 | 62 | | | | 59 |
| | | | | | | 90 | | | | | | | | | – |
| | | 0 | 5 | | | 100 | | 100 | | 0 | | 97 | 100 | 100 | 90 |
| | | 51 | 57 | | | 99 | 86 | 100 | | | | 96 | | | 84 |
| | MEAN | 47 | 47 | 80 | 100 | 98 | 76 | 99 | 14 | 17 | 62 | 97 | 100 | 100 | 78 |
| B | 0.100 | 40 | 43 | 80 | 90 | 83 | | | | | | | | | 42 |
| | | | 42 | 14 | | 30 | 88 | 4 | 44 | 5 | 19 | 39 | | | 33 |
| | | | | | | 100 | | | | | | | | | – |
| | | 0 | 42 | | | 100 | | 99 | | 23 | | 23 | 100 | 100 | 30 |
| | | 22 | 14 | | | 88 | 83 | 71 | | | | 32 | | | 43 |
| | MEAN | 26 | 28 | 80 | 60 | 92 | 44 | 71 | 5 | 21 | 39 | 28 | 100 | 100 | 37 |

## Claims

1. Phenoxy phthalide derivatives having the general formula II:

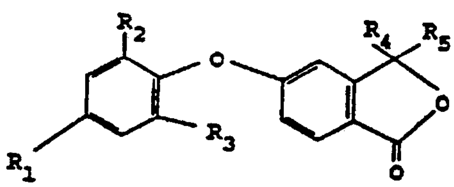

(II)

wherein $R_1$ represents a hydrogen or halogen atom or an alkyl or haloalkyl group; $R_2$ end $R_3$, which may be the same or different, each independently represents a hydrogen or halogen atom or an alkyl, haloalkyl, nitro or cyano group; and $R_4$ end $R_5$, which may be the same or different, each independently represents an alkyl group.

2. Compounds as claimed in claim 1 wherein $R_1$ represents a halogen atom or trifluoromethyl group; $R_2$ represents a halogen atom, or a nitro, cyano, or trifluoromethyl group; end $R_3$ represents a hydrogen or halogen atom.

3. Compounds as claimed in claim 2 wherein $R_1$ represent a trifluoromethyl group; $R_2$ represents a halogen atom; and $R_3$ represents a hydrogen atom.

4. Compounds as claimed in claim 1, 2 or 3 wherein the groups $R_4$ end $R_5$ each independently represents an alkyl group containing 1 - 6 carbon atoms.

5. Compounds as claimed in claim 4, wherein $R_4$ and $R_5$ each independently represent a methyl group or an ethyl group.

6. Compounds as defined in claim 1 and specifically named in Examples 1, 2, 3 or 4 herein.

7. A process for the preparation of a phenoxy phthalide derivative of formula II as claimed in any of claims 1 to 6, which comprises reacting a compound of formula III

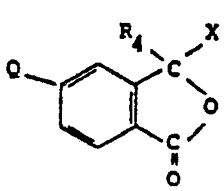

(III)

where X represents a hydrogen atom, a hydroxy group or a complex organometallic alkoxide group, Q represents a halogen atom or a nitro group or a group of formula -OZ, where Z represents a hydrogen or alkali metal atom or a group of formula

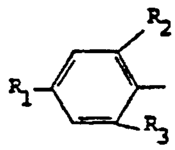

with a compound of formula $R^5$-$(M)_p$-Hal (IV) where M represents a metal atom, p is O (when X is hydrogen) or 1 (when X is other than hydrogen) and Hal represents a halogen atom, and, when the product is a compound in which Q does not represent a group of formula

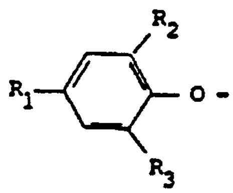

reacting said compound with a compound of formula V

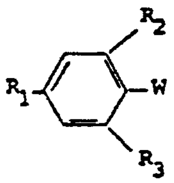

(V)

in which compounds W represents a halogen atom or a nitro group when Q represents a group -OZ where Z represents a hydrogen or alkali metal atom, and W represents such a group -OZ when Q represents a halogen atom or a nitro group.

8. A process as claimed in claim 7, wherein, when p is O, the reaction with the compound of formula IV is carried out in the presence of a base and an aprotic solvent, and, when p is 1, the reaction with the compound of formula IV is carried out in an inert organic solvent in the substantial absence of water.

9. Process as claim in claim 7 carried out substantially as hereinbefore described with particular reference to Examples 1, 2, 3 or 4.

10. A compound as defined in claim 1, whenever prepared by a process as claimed in claim 7, 8 or 9.

11. Herbicidal composition, which comprises a compound as claimed in any of claims 1 to 6 and 10, together with a carrier.

12. A composition as claimed in claim 11, which comprises at least two carriers, at least one of which is a surface-active agent.

13. Method of combating undesired plant growth at a locus, which comprises treating the locus with a compound as claimed in any one of claims 1 to 6 and 10, or a composition as claimed in either of claims 11 and 12.

14. The use of a compound as claimed in any one of claims 1 to 6 and 10 as a herbicide.

**Claims** (for the Contracting State AT)

1. Herbicidal composition, which comprises a phenoxy phthalide derivative having the general formula II:

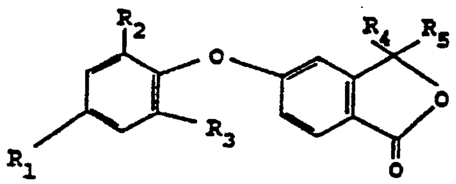

(II)

wherein $R_1$ represents a hydrogen or halogen atom or an alkyl or haloalkyl group; $R_2$ and $A_3$, which may be the same or different, each independently represents a hydrogen or halogen atom or an alkyl, haloalkyl, nitro or cyano group; and $R_4$ and $R_5$, which may be the same or different, each independently represents an alkyl group; together with a carrier.

2. A composition as claimed in claim 1, wherein in the general formula (II) $R_1$ represents a halogen atom or trifluoromethyl group; $R_2$ represents a halogen atom, or a nitro, cyano, or trifluoromethyl group; and $R_3$ represents a hydrogen or halogen atom.

3. A composition as claimed in claim 2, wherein in the general formula II $R_1$ represents a trifluoromethyl group; $R_2$ represents a halogen atom; and $R_3$ represents a hydrogen atom.

4. A composition as claimed in claim 1, 2 or 3, wherein in the general formula II the groups $R_4$ and $R_5$ each independently represents an alkyl group containing 1 - 6 carbon atoms.

5. A composition as claimed in claim 4, wherein in the general formula II $R_4$ and $R_5$ each independently represents a methyl group or an ethyl group.

6. A composition as claimed in claim 1, which comprises a compound of the general formula II as defined in claim 1 and specifically named in Examples 1, 2, 3 or 4 herein, together with a carrier.

7. A composition as claimed in any one of claims 1 to 6, which comprises at least two carriers, at least one of which is a surface-active agent.

8. A process for the preparation of a phenoxy phthalide derivative of formula II as defined in any of claims 1 to 6, which comprises reacting a compound of formula III

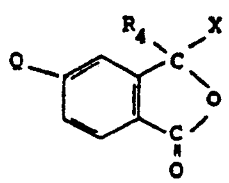

(III)

where X represents a hydrogen atom, a hydroxy group or a complex organometallic alkoxide group, Q represents a halogen atom or a nitro group or a group of formula -OZ, where Z represents a hydrogen or alkali metal atom or a group of formula

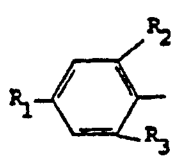

with a compound of formula $R^5$-$(M)_p$-Hal (IV) where M represents a metal atom, p is O (when X is hydrogen) or 1 (when X is other than hydrogen) and Hal represents a halogen atom, and, when the product is a compound in which Q does not represent a group of formula

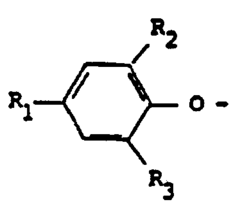

reacting said compound with a compound of formula V

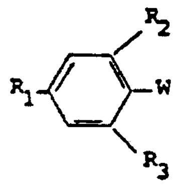

(V)

in which compound W represents a halogen atom or a nitro group when Q represents a group -OZ where Z represents a hydrogen or alkali metal atom, and W represents such a group -OZ when Q represents a halogen atom or a nitro group.

9. A process as claimed in claim 8, wherein, when p is O, the reaction with the compound of formula IV is carried out in the presence of a base and an aprotic solvent, and, when p is 1, the reaction with the compound of formula IV is carried out in an inert organic solvent in the substantial absence of water.

10. Process as claimed in claim 8, carried out substantially as hereinbefore described with particular reference to Examples 1, 2, 3 or 4.

11. Method of combating undesired plant growth at a locus, which comprises treating the locus with a a composition as claimed in any one of claims 1 to 7.

12. The use of a composition as defined in any one of claims 1 to 7, as a herbicide.

**Patentansprüche** (für die Vertragsstaaten BE CH DE FR GB IT LI LU NL SE)

1. Phenoxyphthalidderivate mit der allgemeinen Formel II:

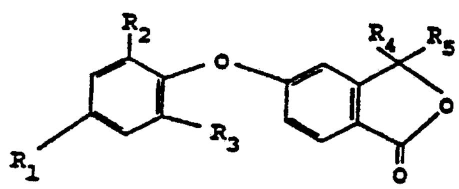

worin $R_1$ ein Wasserstoff- oder Halogenatom oder eine Alkyl- oder Halogenalkylgruppe bedeutet; $R_2$ und $R_3$, die gleich oder verschieden sein können, jeweils unabhängig voneinander ein Wasserstoff- oder Halogenatom oder eine Alkylgruppe, Halogenalkylgruppe, eine Nitro- oder Cyanogruppe bedeuten; und $R_4$ und $R_5$, die gleich oder verschieden sein können, jeweils unabhängig voneinander eine Alkylgruppe darstellen.

2. Verbindungen nach Anspruch 1, worin $R_1$ ein Halogenatom oder eine Trifluormethylgruppe bedeutet; $R_2$ ein Halogenatom oder eine Nitro-, Cyano- oder Trifluormethylgruppe darstellt; und $R_3$ ein Wasserstoff- oder Halogenatom bedeutet.

3. Verbindungen nach Anspruch 2, worin $R_1$ eine Trifluormethylgruppe darstellt; $R_2$ ein Halogenatom bedeutet; und $R_3$ ein Wasserstoffatom darstellt.

4. Verbindungen nach Anspruch 1, 2 oder 3, worin die Gruppen $R_4$ und $R_5$ jeweils unabhängig voneinander eine 1 - 6 Kohlenstoffatome enthaltende Alkylgruppe bedeuten.

5. Verbindungen nach Anspruch 4, worin $R_4$ und $R_5$ jeweils unabhängig voneinander eine Methylgruppe oder eine Ethylgruppe bedeuten.

6. Verbindungen gemäß Definition in Anspruch 1 und speziell angeführt in den vorliegenden Beispielen 1, 2, 3 oder 4.

7. Verfahren zur Herstellung eines Phenoxyphthalidderivats der Formel II, wie in einem der Ansprüche 1 bis 6 beansprucht, welches ein Umsetzen einer Verbindung der Formel III

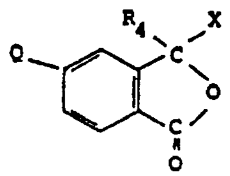

(III)

worin X ein Wasserstoffatom, eine Hydroxygruppe oder eine komplexe organometallische Alkoxidgruppe bedeutet, Q ein Halogenatom oder eine Nitrogruppe oder eine Gruppe der Formel -OZ darstellt, worin Z ein Wasserstoff- oder Alkalimetallatom oder eine Gruppe der Formel

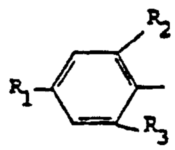

bedeutet, mit einer Verbindung der Formel $R^5$-$(M)_p$-Hal (IV), worin M ein Metallatom darstellt, p für O steht (wenn X Wasserstoff bedeutet) oder für 1 steht (wenn X eine von Wasserstoff verschiedene Bedeutung hat) und Hal ein Halogenatom darstellt, und, falls das Produkt eine Verbindung ist, worin Q nicht eine Gruppe der Formel

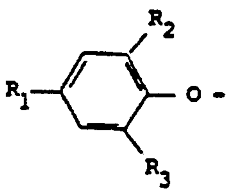

bedeutet, ein Umsetzen dieser Verbindung mit einer Verbindung der Formel V

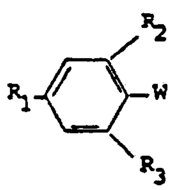

(V)

umfaßt, in welcher Verbindung W ein Halogenatom oder eine Nitrogruppe darstellt, wenn Q eine Gruppe -OZ bedeutet, worin Z ein Wasserstoffatom oder ein Alkalimetallatom bedeutet, und worin W eine solche Gruppe -OZ darstellt, wenn Q ein Halogenatom oder eine Nitrogruppe bedeutet.

8. Verfahren nach Anspruch 7, worin, wenn p den Wert O hat, die Umsetzung mit der Verbindung der Formel IV in Gegenwart einer Base und eines aprotischen Lösungsmittels ausgeführt wird, und, wenn p den Wert 1 hat, die Umsetzung mit der Verbindung der Formel IV in einem inerten organischen Lösungsmittel bei im wesentlichen Abwesenheit von Wasser ausgeführt wird.

9. Verfahren nach Anspruch 7, das im wesentlichen wie zuvor beschrieben ausgeführt wird, unter spezieller Bezugnahme auf die Beispiele 1, 2, 3 oder 4.

10. Eine Verbindung gemäß Definition in Anspruch 1, wannimmer sie nach einem Verfahren wie in Anspruch 7, 8 oder 9 beansprucht hergestellt ist.

11. Herbizide Zusammensetzung, welche eine Verbindung, wie in einem der Ansprüche 1 bis 6 und 10 beansprucht, zusammen mit einem Träger umfaßt.

12. Eine Zusammensetzung, wie in Anspruch 11 beansprucht, welche wenigstens zwei Träger umfaßt, von denen wenigstens einer ein oberflächenaktives Mittel ist.

13. Verfahren zur Bekämpfung von unerwünschtem Pflanzenwuchs an einem Ort, welches ein Behandeln des Ortes

mit einer Verbindung, wie in einem der Ansprüche 1 bis 6 und 10 beansprucht, oder mit einer Verbindung, wie in Anspruch 11 oder 12 beansprucht, umfaßt.

14. Verwendung einer Verbindung, wie in einem der Ansprüche 1 bis 6 und 10 beansprucht, als Herbizid.

**Patentansprüche** (für den Vertragsstaat AT)

1. Herbizide Zusammensetzung, welche ein Phenoxyphthalidderivat mit der allgemeinen Formel II:

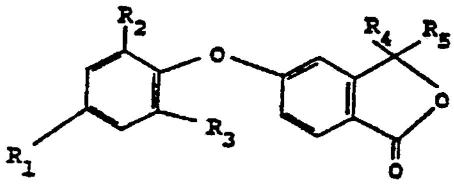

(II)

worin $R_1$ ein Wasserstoff- oder Halogenatom oder eine Alkyl- oder Halogenalkylgruppe bedeutet; $R_2$ und $R_3$, die gleich oder verschieden sein können, jeweils unabhängig voneinander ein Wasserstoff- oder Halogenatom oder eine Alkylgruppe, Halogenalkylgruppe, eine Nitro- oder Cyanogruppe bedeuten; und $R_4$ und $R_5$, die gleich oder verschieden sein können, jeweils unabhängig voneinander eine Alkylgruppe darstellen, zusammen mit einem Träger umfaßt.

2. Zusammensetzung nach Anspruch 1, worin in der allgemeinen Formel (II) $R_1$ ein Halogenatom oder eine Trifluormethylgruppe bedeutet; $R_2$ ein Halogenatom oder eine Nitro-, Cyano- oder Trifluormethylgruppe darstellt; und $R_3$ ein Wasserstoff- oder Halogenatom bedeutet.

3. Zusammensetzung nach Anspruch 2, worin in der allgemeinen Formel II $R_1$ eine Trifluormethylgruppe darstellt; $R_2$ ein Halogenatom bedeutet; und $R_3$ ein Wasserstoffatom darstellt.

4. Zusammensetzung nach Anspruch 1, 2 oder 3, worin in der allgemeinen Formel II die Gruppen $R_4$ und $R_5$ jeweils unabhängig voneinander eine 1 - 6 Kohlenstoffatome enthaltende Alkylgruppe bedeuten.

5. Zusammensetzung nach Anspruch 4, worin in der allgemeinen Formel II $R_4$ und $R_5$ jeweils unabhängig voneinander eine Methylgruppe oder eine Ethylgruppe bedeuten.

6. Zusammensetzung nach Anspruch 1, welche eine Verbindung der allgemeinen Formel II, wie in Anspruch 1 definiert, und speziell genannt in den vorliegenden Beispielen 1, 2, 3 oder 4, zusammen mit einem Träger umfaßt.

7. Zusammensetzung nach einem der Ansprüche 1 bis 6, welche wenigstens zwei Träger, von denen wenigstens einer ein oberflächenaktives Mittel ist, umfaßt.

8. Verfahren zur Herstellung eines Phenoxyphthalidderivats der Formel II, wie in einem der Ansprüche 1 bis 6 beansprucht, welches ein Umsetzen einer Verbindung der Formel III

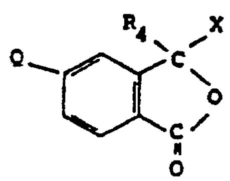

(III)

worin X ein Wasserstoffatom, eine Hydroxygruppe oder eine komplexe organometallische Alkoxidgruppe bedeutet, Q ein Halogenatom oder eine Nitrogruppe oder eine der Formel -OZ darstellt, worin Z ein Wasserstoff- oder Alkalimetallatom oder eine Gruppe der Formel

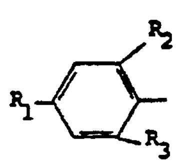

bedeutet, mit einer Verbindung der Formel $R^5$-$(M)_p$-Hal (IV), worin M ein Metallatom darstellt, p für O steht (wenn X Wasserstoff bedeutet) oder für 1 steht (wenn X eine von Wasserstoff verschiedene Bedeutung hat) und Hal ein Halogenatom darstellt, und, falls das Produkt eine Verbindung ist, worin Q nicht eine Gruppe der Formel

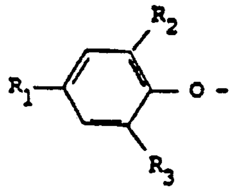

bedeutet, ein Umsetzen dieser Verbindung mit einer Verbindung der Formel V

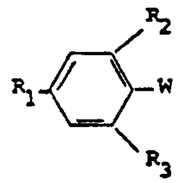

(V)

umfaßt, in welcher Verbindung W ein Halogenatom oder eine Nitrogruppe darstellt, wenn Q eine Gruppe -OZ bedeutet, worin Z ein Wasserstoffatom oder ein Alkalimetallatom bedeutet, und worin W eine solche Gruppe -OZ darstellt, wenn Q ein Halogenatom oder eine Nitrogruppe bedeutet.

9. Verfahren nach Anspruch 8, worin, wenn p den Wert O hat, die Umsetzung mit der Verbindung der Formel IV in Gegenwart einer Base und eines aprotischen Lösungsmittels ausgeführt wird, und, wenn p den Wert 1 hat, die Umsetzung mit der Verbindung der Formel IV in einem inerten organischen Lösungsmittel bei im wesentlichen Abwesenheit von Wasser ausgeführt wird.

10. Verfahren nach Anspruch 8, das im wesentlichen wie zuvor beschrieben ausgeführt wird, unter spezieller Bezugnahme auf die Beispiele 1, 2, 3 oder 4.

11. Verfahren zur Bekämpfung von unerwünschtem Pflanzenwuchs an einem Ort, welches ein Behandeln des Ortes mit einer Verbindung, wie in einem der Ansprüche 1 bis 6 definiert, oder mit einer Zusammensetzung, wie in einem der Ansprüche 1 bis 7 beansprucht, umfaßt.

12. Verwendung einer Verbindung, wie in einem der Ansprüche 1 bis 6 definiert, als Herbizid.

**Revendications** (BE CH DE FR GB IT LU NL SE)

1. Dérivés de phtalures phénoxylés présentant la formule générale II:

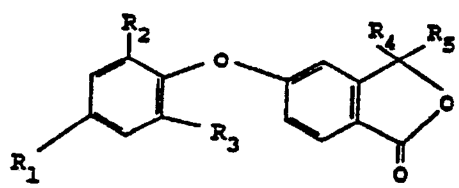

(II)

dans laquelle $R_1$ représente un atome d'hydrogène ou d'halogène ou un groupe alkyle ou haloalkyle; $R_2$ et $R_3$ qui peuvent être identiques ou différents, représentent chacun indépendamment un atome d'hydrogène ou d'halogène ou un groupe alkyle, haloalkyle, nitro ou cyano; et $R_4$ et $R_5$, qui peuvent être identiques ou différents, représentent chacun indépendamment un groupe alkyle.

2. Composés tels que revendiqués dans la revendication 1, dans lesquels $R_1$ représente un atome d'halogène ou un groupe trifluorométhyle; $R_2$ représente un atome d'halogène, ou un groupe nitro, cyano ou trifluorométhyle; et $R_3$ représente un atome d'hydrogène ou d'halogène.

3. Composés tels que revendiqués dans la revendication 2, dans lesquels $R_1$ représente un groupe trifluorométhyle; $R_2$ représente un atome d'halogène; et $R_3$ représente un atome d'hydrogène.

4. Composés tels que revendiqués dans la revendication 1, 2 ou 3, dans lesquels les groupes $R_4$ et $R_5$ représentent chacun indépendamment un groupe alkyle renfermant de 1 à 6 atomes de carbone.

5. Composés tels que revendiqués dans la revendication 4, dans lesquels $R_4$ et $R_5$ représentent chacun indépendamment un groupe méthyle ou un groupe éthyle.

6. Composés tels que définis dans la revendication 1 et cités plus spécifiquement dans les présents exemples 1, 2, 3 ou 4.

7. Un procédé pour la préparation d'un dérivé de phtalure phénoxylé de formule II, tel que revendiqué dans l'une quelconque des revendications 1 à 6, qui consiste à faire réagir un composé de formule III

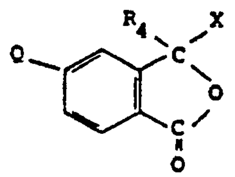

(III)

dans laquelle X représente un atome d'hydrogène, un groupe hydroxy ou un groupe complexe d'alcoolate organométallique, Q représente un atome d'halogène ou un groupe nitro ou bien un groupe de formule -OZ, dans laquelle Z représente un atome d'hydrogène ou de métal alcalin ou bien un groupe de formule

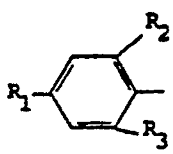

avec un composé de formule $R^5$-$(M)_p$-Hal (IV) dans laquelle M représente un atome métallique, p est O (lorsque X est de l'hydrogène) ou 1 (lorsque X est autre chose que de l'hydrogène) et Hal représente un atome d'halogène, et, lorsque le produit est un composé dans lequel Q ne représente pas un groupe de formule

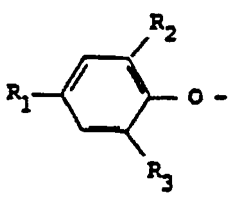

en faisant réagir ledit composé avec un composé de formule V

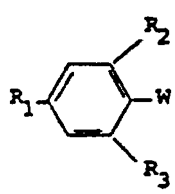

(V)

dans laquelle le composé W représente un atome d'halogène ou un groupe nitro lorsque Q représente un groupe -OZ ou Z représente un atome d'hydrogène ou de métal alcalin, et W représente un tel groupe -OZ lorsque Q représente un atome d'halogène ou un groupe nitro.

8. Un procédé tel que revendiqué dans la revendication 7, dans laquelle, lorsque p est O, la réaction avec le composé de formule IV est réalisée en présence d'une base et d'un solvant aprotique et, lorsque p est 1, la réaction avec le composé de formule IV est réalisée dans un solvant organique inerte pratiquement en l'absence d'eau.

9. Procédé tel que revendiqué dans la revendication 7, réalisé pratiquement comme décrit ci-dessus en se référant particulièrement aux exemples 1, 2, 3 ou 4.

10. Un composé tel que défini dans la revendication 1, lorsqu'il est préparé par un procédé tel que revendiqué dans la revendication 7, 8 ou 9.

11. Composition herbicide, qui comprend un composé tel que revendiqué dans l'une quelconque des revendications 1 à 6 et 10, conjointement avec un support.

12. Une composition telle que revendiquée dans la revendication 11, qui comprend au moins deux supports, dont au moins l'un est un agent tensio-actif.

13. Méthode pour combattre la croissance de plantes indésirables en un site, qui consiste à traiter le site avec un composé tel que revendiqué dans l'une quelconque des revendications 1 à 6 et 10 ou une composition telle que revendiquée dans l'un quelconque des revendications 11 et 12.

14. L'utilisation d'un composé tel revendiqué dans l'une quelconque des revendications 1 à 6 et 10, en tant qu'herbicide.

**Revendications** (pour l'état contractant AT)

1. Composition herbicide, qui comprend un dérivé de phtalure phénoxylé présentant la formule générale II:

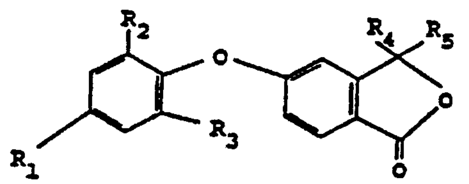

(II)

dans laquelle $R_1$ représente un atome d'hydrogène ou d'halogène ou un groupe alkyle ou haloalkyle; $R_2$ et $R_3$, qui peuvent être identiques ou différents, représentent chacun indépendamment un atome d'hydrogène ou d'halogène ou un groupe alkyle, haloalkyle, nitro ou cyano; et $R_4$ et $R_5$, qui peuvent être identiques ou différents, représentent chacun indépendamment un groupe alkyle; conjointement avec un support.

2. Une composition telle que revendiquée dans la revendication 1, dans laquelle, dans la formule générale (II), $R_1$ représente un atome d'halogène ou un groupe trifluorométhyle; $R_2$ représente un atome d'halogène ou un groupe nitro, cyano ou trifluorométhyle; et $R_3$ représente un atome d'hydrogène ou d'halogène.

3. Une composition telle que revendiquée dans la revendication 2, dans laquelle, dans la formule générale II, $R_1$ représente un groupe trifluorométhyle; $R_2$ représente un atome d'halogène; et $R_3$ représente un atome d'hydrogène.

4. Une composition telle que revendiquée dans la revendication 1, 2 ou 3, dans laquelle, dans la formule générale II, les groupes $R_4$ et $R_5$ représentent chacun indépendamment un groupe alkyle renfermant de 1 à 6 atomes de carbone.

5. Une composition telle que revendiquée dans la revendication 4, dans laquelle, dans la formule générale II, $R_4$ et $R_5$ représentent chacun indépendamment un groupe méthyle ou un groupe éthyle.

6. Une composition telle que revendiquée dans la revendication 1, qui comprend un composé de formule générale II telle que défini dans la revendication 1 et spécifiquement désigné dans les présents exemples 1, 2 ou 3 ou 4, conjointement avec un support.

7. Une composition telle que revendiquée dans l'une quelconque des revendications 1 à 6, qui comprend au moins deux supports dont au moins un est un agent tensio-actif.

8. Un procédé pour la préparation d'un dérivé de phtalure phénoxylé de formule II tel que défini dans l'une quelconque des revendications 1 à 6, qui consiste à faire réagir un composé de formule III

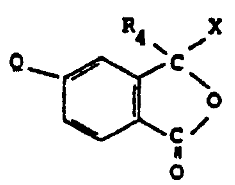

(III)

dans laquelle X représente un atome d'hydrogène, un groupe hydroxy ou un groupe complexe d'alcoolate organométallique, Q représente un atome d'halogène ou un groupe nitro ou bien un groupe de formule -OZ, ou Z représente un atome d'hydrogène ou de métal alcalin ou bien un groupe de formule

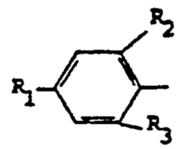

avec un composé de formule $R^5$-$(M)_p$-Hal (IV) dans laquelle M représente un atome métallique, p est O (lorsque X est de l'hydrogène) ou 1 (lorsque X est autre chose que de l'hydrogène) et Hal représente un atome d'halogène, et, lorsque le produit est un composé dans lequel Q ne représente pas un groupe de formule

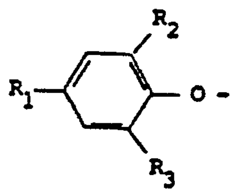

en faisant réagir ledit composé avec un composé de formule V

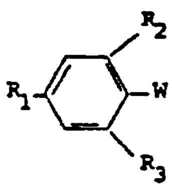

(V)

composé dans lequel W représente un atome d'halogène ou un groupe nitro lorsque Q représente un groupe -OZ dans lequel Z représente un atome d'hydrogène ou de métal alcalin, et W représente un tel groupe -OZ lorsque Q représente un atome d'halogène ou un groupe nitro.

9. Un procédé tel que revendiqué dans la revendication 8, dans lequel, lorsque p est O, la réaction avec le composé de formule IV est réalisée en présence d'une base et d'un solvant aprotique, et, lorsque p est 1, la réaction avec le composé de formule IV est réalisée dans un solvant organique inerte pratiquement en l'absence d'eau.

10. Procédé tel que revendiqué dans la revendication 8, réalisé pratiquement comme décrit ci-dessus en se référant particulièrement aux exemples 1, 2, 3 ou 4.

11. Méthode pour combattre la croissance des plantes indésirables en un site, qui comprend le traitement du site avec un composé tel que définit dans l'une quelconque des revendications 1 à 6 ou une composition telle que revendiquée dans l'une quelconque des revendications 1 à 7.

12. L'utilisation d'un composé tel que défini dans l'une quelconque des revendications 1 à 6, comme herbicide.